(19) Europäisches Patentamt
European Patent Office
Office européen des brevets

(11) Veröffentlichungsnummer: **0 548 897 A2**

(12) **EUROPÄISCHE PATENTANMELDUNG**

(21) Anmeldenummer: **92121746.9**

(51) Int. Cl.[5]: **C07C 239/10**, C07C 211/35

(22) Anmeldetag: **21.12.92**

(30) Priorität: **24.12.91 DE 4142975**

(43) Veröffentlichungstag der Anmeldung:
**30.06.93 Patentblatt 93/26**

(84) Benannte Vertragsstaaten:
**CH DE FR GB LI**

(71) Anmelder: **EMS-INVENTA AG**
**Selnaustrasse 16**
**CH-8001 Zürich(CH)**

(72) Erfinder: **Stoss, Peter, Dr. rer. nat.**
**Mozartstrasse 15**
**W-7918 Illertissen(DE)**

(54) **Cyclohexanon-oxim-Derivate, Verfahren zu ihrer Herstellung sowie Verfahren zu ihrer Weiterverarbeitung zu Cyclohexanon- und Cyclohexylamin-Derivaten.**

(57) Die Erfindung betrifft Alkyl- oder cycloalkylsubstituierte Cyclohexanon-oxime, wobei das Alkyl- oder Cycloalkyl eine geradkettige oder verzweigte Alkylgruppe mit 1 bis 10 C-Atomen oder eine Cycloalkylgruppe mit 3 bis 10 C-Atomen ist und worin die Bindung des Cyclohexyl-Ringes in Bezug zum Substituenten R sowohl cis- als auch trans-konfiguriert sein kann sowie ihre Weiterverarbeitung zu hochreinen Cyclohexanon-Derivaten und zu hochreinen Cyclohexylamin-Derivaten.

EP 0 548 897 A2

Die Erfindung betrifft den in den Patentansprüchen angegebenen Gegenstand. Die Erfindung betrifft insbesondere neue Cyclohexanon-oxim-Derivate der allgemeinen Formel I:

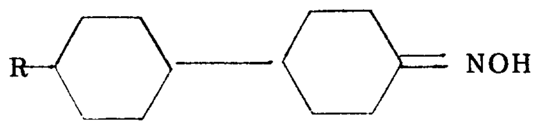

worin R eine geradkettige oder verzweigte Alkylgruppe mit 1 bis 10 C-Atomen oder eine Cycloalkylgruppe mit 3 bis 10 C-Atomen ist und worin die Bindung des zweiten Cyclohexyl-Ringes in Bezug zum Substituenten R sowohl cis- als auch trans-konfiguriert sein kann.

Die Erfindung betrifft weiterhin ein Verfahren zur Herstellung dieser Cyclohexanon-oxim-Derivate sowie ihre Weiterverarbeitung zu hochreinen Cyclohexanon-Derivaten der allgemeinen Formel II:

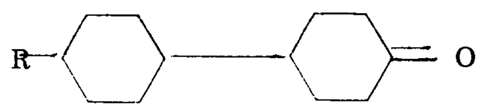

worin R die in den Ansprüchen 1 bis 5 angegebene Bedeutung hat und die Bindung des Cyclohexyl-Ringes in Bezug zum Substituenten R cis- oder trans-konfiguriert sein kann oder zu hochreinen Cyclohexylamin-Derivaten der weiter unten angegebenen allgemeinen Formel III.

Die Erfindung betrifft weiterhin die durch dieses Verfahren hergestellten neuen Cyclohexylamin-Derivate der folgenden allgemeinen Formel III:

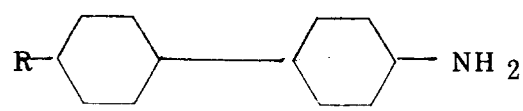

worin R eine geradkettige oder verzweigte Alkylgruppe mit 1, 2 oder 4 bis 10 C-Atomen oder eine Cycloalkylgruppe mit 3 bis 10 C-Atomen ist und die Bindung des zweiten Cyclohexyl-Ringes in Bezug zum Substituenten R cis- oder trans-konfiguriert sein kann, sowie deren Salze mit anorganischen oder organischen Säuren.

In den Unteransprüchen sind vorteilhafte Ausführungsformen der Erfindung enthalten.

Cyclohexanon-Derivate der allgemeinen Formel II können nach an sich bekannten Methoden hergestellt werden, wie sie in der Literatur (z.B. in den Standardwerken wie Houben-Weyl, Methoden der organischen Chemie, Georg Thieme Verlag, Stuttgart) beschrieben sind, und zwar unter Reaktionsbedingungen, die für die genannten Umsetzungen bekannt und geeignet sind.

Bei diesen bekannten und deshalb allgemein angewandten Herstellungsverfahren dieser Ketone mit der allgemeinen Formel II fallen normalerweise Gemische an, die durch übliche, technisch realisierbare Reinigungsmethoden, wie Kristallisation oder Destillation nicht in die Komponenten auftrennbar sind. Vielmehr muss zu aufwendigen, chemischen Reinigungsmethoden gegriffen werden. Als solche sind z.B. bekannt: Derivatisierung der hydroxylgruppenhaltigen Nebenprodukte durch Veresterung mit geeigneten Carbonsäuren und nachfolgende destillative Abtrennung der gebildeten Ester vom Keton. Dabei liegen jedoch häufig auch in diesen Fällen die Siedepunkte der beteiligten Reaktionspartner so eng zusammen, dass keine befriedigende Auftrennung erreichbar ist. Darüberhinaus erfordert die Suche nach der im Einzelfall geeigneten Carbonsäure in der Regel einen erheblichen Zeitaufwand.

Cyclohexanon-Derivate mit der allgemeinen Formel II spielen eine sehr wichtige Rolle, insbesondere als wichtige Zwischenprodukte auf dem Sektor der Flüssigkristalle (siehe z.B. EP 198 714, DE-OS 37 31 619). Für diesen Verwendungszweck werden die Verbindungen II in hoher Reinheit benötigt.

Es ist daher Aufgabe der Erfindung, neue Cyclohexanon-oxim-Derivate als Zwischenprodukte zur Verfügung zu stellen, die zu hochreinen Cyclohexanon-Derivaten mit der Formel II weiterverarbeitet werden können. Weiterhin sollen Verfahren zur Herstellung von hochreinen Verbindungen der Formel II sowie von hochreinen Cyclohexylamin-Derivaten der Formel III aus den hochreinen Cyclohexanon-oxim-Derivaten

angegeben werden.

Diese Aufgaben werden durch Anspruch 1, 6, 9, 10 gelöst, während Anspruch 12 die aus den neuen Cyclohexanon-oximen hergestellten neuen Cyclohexylamin-Derivate betrifft.

Die erfindungsgemässen Cyclohexanon-oxim-Derivate haben die allgemeine Formel I, worin R eine geradkettige oder verzweigte Alkylgruppe mit 1 bis 10 C-Atomen oder eine Cycloalkylgruppe mit 3 bis 10 C-Atomen ist und worin die Bindung des zweiten Cyclohexyl-Ringes in Bezug zum Substituenten R sowohl cis-, als auch trans-konfiguriert sein kann. Besonders bevorzugte Substituenten R sind dabei geradkettige und verzweigte Alkylgruppen mit 1 bis 7 Kohlenstoffatomen, sowie Cycloalkylgruppen mit 4 bis 7 C-Atomen, insbesondere Methyl-Ethyl-, Propyl-, Butyl-, Pentyl-, Hexyl-, Heptyl-Reste, Isopropyl-, Isobutyl-, Isopentyl-, Isohexyl- und Isoheptyl-Reste in allen möglichen isomeren Formen, sowie Cyclobutyl-, Cyclopentyl-, Cyclohexyl- und Cycloheptyl-Reste. Die transkonfigurierten isomeren Formen sind dabei besonders bevorzugt.

Die erfindungsgemässen Verbindungen der allgemeinen Formel I können zur Herstellung von hochreinen Cyclohexanon-Derivaten der allgemeinen Formel II, worin R die oben angegebene Bedeutung besitzt und die Bindung des zweiten Cyclohexyl-Ringes in Bezug zu Substituenten R sowohl cis- als auch transkonfiguriert sein kann, sowie zur Herstellung von hochreinen Cyclohexylamin-Derivaten der allgemeinen Formel III eingesetzt werden, worin R die oben angegebene Bedeutung besitzt und die Bindung des zweiten Ringes in Bezug zum Substituenten R sowohl cis- als auch trans-konfiguriert sein kann, sowie deren Salzen mit anorganischen und organischen Säuren.

Darüber hinaus betrifft die vorliegende Erfindung auch Verfahren zur Herstellung von hochreinen Cyclohexanon Derivaten der allgemeinen Formel II, dadurch charakterisiert, dass die neuen Cyclohexanon-oxim-Derivate der allgemeinen Formel I, auf an sich bekannte Weise, zu den hochreinen Cyclohexanon Derivaten der allgemeinen Formel II gespalten werden, sowie Verfahren zur Herstellung von neuen, hochreinen Cyclohexylamin Derivaten der allgemeinen Formel III und deren Salzen mit anorganischen und organischen Säuren, dadurch gekennzeichnet, dass die neuen Cyclohexanon-oxim-Derivate der allgemeinen Formel I, auf an sich bekannte Weise, zu den, mit Ausnahme von R = Propyl, neuen Cyclohexylamin Derivaten der allgemeinen Formel III reduziert werden.

Mit der Bereitstellung der erfindungsgemässen hochreinen Verbindungen der Formel I und der nachfolgenden Weiterverarbeitung derselben in die Verbindungen II kann die hohe Reinheitsforderung für Flüssigkristalle erstmals mit vertretbarem Zusatzaufwand gewährleistet werden. Damit erfolgt gleichzeitig die Befriedigung eines technischen Bedürfnisses, was bisher auf anderem Weg nicht möglich war.

Die erfindungsgemässen Cyclohexylamin-Derivate III können, als sterisch anspruchsvolle und speziell substituierte Amine insbesondere für die Herstellung von Pharmazeutika, Farbstoffen, und ganz besonders auch auf dem Sektor der Flüssigkristalle, aber auch für Agrochemikalien der Kunststoffe Einsatz finden. Die ganze Einsatzbreite der erfindungsgemässen hochreinen Derivate der Formel III konnte bisher noch nicht ausgeschöpft werden, da mit einer einzigen Ausnahme, wobei R = Propyl ist, derartige Verbindungen bisher nicht beschrieben sind. Sie stellen somit ebenfalls neuartige Strukturen dar. Die einzige bekannte Ausnahme (R = Propyl) ist in der Offenlegungsschrift JP 01 26 552 erwähnt und wird dort als Reaktionspartner zur Herstellung von Flüssigkristallen eingesetzt.

Neben den freien Aminen der Formel III können auch deren Salze mit anorganischen und organischen Säuren hergestellt werden. Als Salze seien beispielhaft aufgeführt: Hydrochloride, Phosphate, Sulfate, Nitrate, Tetrafluorborate, Formiate, Acetate, Propionate, Carbonate, Benzonate, Toluolsulfonate, Naphthalinsulfonate, Oxalate, Fumarate, Tartrate, und dergleichen.

Die erfindungsgemässen Verbindungen der allgemeinen Formel I können beispielsweise hergestellt werden, indem man entsprechend substituierte Phenole der allgemeinen Formel IV:

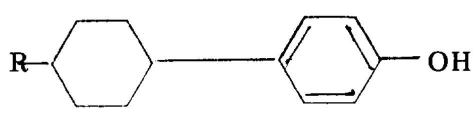

worin R die oben angegebene Bedeutung besitzt und die Bindung des zweiten Ringes in Bezug zum Substituenten R cis- oder trans-konfiguriert sein kann, einer selektiven katalytischen Hydrierung unterwirft.

Dabei erhält man, wie aus der Literatur für ähnliche Fälle bekannt, ein Produktgemisch, bestehend aus wechselnden Anteilen Cyclohexanon-Derivaten II, Cyclohexanol-Derivaten der allgemeinen Formel V:

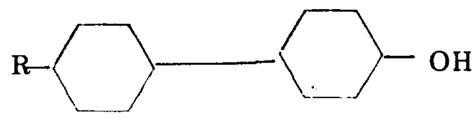

sowie nicht umgesetzten Edukten IV.

Je nach der cis oder trans-konfigurierten Bindung des aromatischen Ringes und des Substituenten R bei den zur Reaktion eingesetzten Phenolen IV besitzen die Produkte II und V ebenfalls cis- oder trans-Konfiguration, wobei in der praktischen Anwendung im allgemeinen die trans-Verbindungen bevorzugt werden.

Die Produktverteilung bei dieser katalytischen Hydrierung ist zwar durch die angewandten Reaktionsbedingungen in gewissen Grenzen beeinflussbar. Durch optimale Steuerung von Druck, Temperatur, Zeit, Lösungsmittel, Verdünnungsgrad, Katalysator-Art und -Menge kann immerhin eine bevorzugte Bildung des gewünschten Ketons II erreicht werden. Jedoch ist bisher keine Verfahrensvariante bekannt, bei der die möglichen Nebenprodukte IV und V in Anteilen unter 10 bis 15% entstehen. Alle im Gemisch vorliegenden Substanzen stellen im allgemeinen Feststoffe dar, die sich hinsichtlich ihrer Löslichkeiten in verschiedenen Lösungsmitteln und in anderen physikalischen Eigenschaften nicht deutlich genug unterscheiden, um eine einwandfreie Trennung zu ermöglichen. Versuche, eine Reinigung durch grosstechnisch anwendbare Verfahren, wie Umkristallisation oder Destillation zu erreichen, blieben deshalb auch erfolglos. Chromatografische Trennverfahren, die im Labor problemlos anwendbar sind, können aus wirtschaftlichen und verfahrenstechnischen Gründen nicht in den Produktionsmassstab übertragen werden.

Es bestand daher ein dringendes Bedürfnis nach einem mit vertretbarem Aufwand durchführbaren Isolierungs- und Reinigungsverfahren für die begehrten hochreinen Ketone II.

Durch Ueberführung der im rohen Reaktionsgemisch vorliegenden Ketone II in die erfindungsgemässen neuen Oxime I gelingt eine überraschend einfache Trennung von den anderen vorhandenen Begleitstoffen. Da die Oxime I Feststoffe darstellen, die unter den gewählten Reaktionsbedingungen schwer löslich sind, lassen sie sich in hoher Reinheit von den in den Mutterlaugen in Lösung verbleibenden übrigen Begleitstoffen abtrennen. Gegebenenfalls können die auf diese Weise isolierten Oxime I einer zusätzlichen Reinigung unterworfen werden.

Die Abtrennung von Ketonen aus Produktgemischen in Form ihrer Oxime stellt zwar eine gelegentlich durchgeführte Arbeitsweise dar. Jedoch sind für Erfolg oder Scheitern derartige Operationen immer das Verhalten und die Eigenschaften der Ketone selbst sowie der anderen im Gemisch vorhandenen Stoffe entscheidend. Wegen der strukturellen Aehnlichkeit der in diesem Falle beteiligten Komponenten war eine Trennung auf derartige Weise jedoch nicht so ohne weiteres zu erwarten. In der Tat sind bisher auch keinerlei derartige Versuche mit den beschriebenen Verbindungen bekannt geworden. Der Versuch, Oxime als Schutzgruppen von Ketonen einzusetzen und zur Trennung von anderen Nebenprodukten zu verwenden, ist zwar im Prinzip ebenfalls bekannt, jedoch nicht besonders attraktiv. Nach Houben-Weyl, Band 7/2b, S. 1897 ff wird von dieser Möglichkeit mit der Feststellung abgeraten, dass die Oxime nur schwer und in schlechten Ausbeuten in Ketone rückspaltbar sind. Dass genau dies im vorliegenden Fall der erfindungsgemässen Oxime I nicht zutrifft, war selbst für einen Fachmann nicht vorhersehbar.

Die Herstellung der erfindungsgemässen Oxime I kann nach bekannten Analogieverfahren erfolgen, wie sie in Houben-Weyl Bd. 10/4, S. 55 ff beschrieben werden. Dazu wird z.B. das nach der katalytischen Hydrierung entsprechender Phenole IV vorliegende Rohgemisch, das die Verbindungen II, IV und V in wechselnden Anteilen enthalten kann, in einem geeigneten Lösungsmittel gelöst und mit Hydroxylamin versetzt. Anstelle von Hydroxylamin können auch dessen Salze mit anorganischen oder organischen Säuren, vorzugsweise Hydroxylamin-sulfat, Hydroxylamin-hydrochlorid, Hydroxylamin-phosphat, Hydroxylamin-acetat zur Anwendung gelangen. In diesen Fällen verwendet man berechnete Mengen geeigneter Basen zur Abstumpfung der bei der Reaktion freiwerdenden Säure. Als Basen kommen infrage: Alkalihydroxide, Carbonate, Hydrogencarbonate, Acetate, Ammoniak oder organische Basen, wie z.B. Pyridin. Als Lösungsmittel für die Umsetzung werden bevorzugt niedere Alkohole, wie Methanol, Ethanol, Propanol und Isopropanol oder Gemische dieser Alkohole mit wechselnden Mengen Wasser eingesetzt. Die Umsetzung kann im Bereich von Raumtemperatur bis zur Siedetemperatur des eingesetzten Lösungsmittels durchgeführt werden. Die Reaktionszeiten betragen wenige Minuten bis wenige Stunden.

Die Isolierung der auf diese Weise gebildeten Oxime erfolgt üblicherweise durch Abtrennung des bei der Reaktion gebildeten Niederschlags mittels Filtration. Das so gewonnene Oxim kann durch geeignete Methoden, wie Umkristallisation, Chromatografie oder Destillation zusätzlich gereinigt werden. Im allgemeinen fällt das Oxim jedoch bereits in ausreichend reiner Form an.

Die Umwandlung der erfindungsgemässen Verbindungen I in die technisch begehrten, hochreinen Ketone II kann ebenfalls in Analogie zu bekannten Verbindungen nach Methoden erfolgen, die jedem Fachmann geläufig sind und die hier deshalb nicht im Detail erläutert werden müssen. Neben anderen Verfahren von untergeordneter Bedeutung, bedient man sich vorteilhafterweise einer sauren Hydrolyse, einer oxidativen Spaltung oder der Verdrängung durch reaktionfähigere Carbonylgruppen (Siehe Houben-Weyl Bd. 7/2a, S. 799 ff). Im Gegensatz zu der oben zitierten Lehrmeinung verläuft die Rückspaltung der erfindungsgemässen Oxime I zu den Ketonen II überraschenderweise ohne Probleme und mit guten bis sehr guten Ausbeuten.

Die aus den Oximen I somit in hoher Reinheit zugänglichen Ketone können gegebenenfalls zusätzlich durch Kristallisation, Destillation oder chromatografische Verfahren noch weiter gereinigt werden.

Die Ueberführung der erfindungsgemässen, neuen Oxime I in die, mit einer oben zitierten Ausnahme ebenfalls neuen Cyclohexylamine III, kann in Analogie zu bekannten Methoden erfolgen. Z.B. sind sämtliche in Houben-Weyl Bd.11/1, S. 495 ff aufgeführten Reduktionsmittel und Methoden dafür geeignet.

Beispielsweise kann eine katalytische Hydrierung der Oxime I durchgeführt werden. Dabei entstehen die, bis auf eine Ausnahme (R = Propyl), ebenfalls neuen Cyclohexylamin-Derivate III. Die Verbindungen III können einerseits als solche isoliert und gegebenenfalls weiter gereinigt werden. Falls erforderlich, ist jedoch auch eine Ueberführung in Salze mit anorganischen oder organischen Säuren möglich, die dann in dieser Form isoliert und gegebenenfalls weiter gereinigt werden können.

Mit der Bereitstellung der erfindungsgemässen Amine III eröffnet sich die Möglichkeit des Einsatzes dieser sterisch anspruchsvollen und mit einer zu vielfältigen chemischen Reaktionen befähigten $NH_2$-Gruppe in den verschiedenartigsten Anwendungsgebieten. Beispielsweise können sie als Zwischenprodukte und Reaktionspartner für die Herstellung von Pharmazeutika, Agrochemikalien, Farbstoffen, Kunststoffen, Flüssigkristallen und in anderen Einsatzgebieten genutzt werden.

Die nachfolgenden Beispiele dienen der Erläuterung der Erfindung ohne diese jedoch zu beschränken.

## Beispiele

### Beispiel 1

4-(trans-4-Butylcyclohexyl)-cyclohexanon-oxim

a) 4-(trans-4-Butylcyclohexyl)-cyclohexanon (Rohprodukt)

In einen 1 l VA-Autoklaven werden 100 g 4-(trans-4-Butylcyclohexyl)-phenol und 450 ml tert. Butanol eingefüllt. Das Gemisch wird mit 4,0 g Palladium-Kohle (5%) und 0,5 ml 2 N Natriumacetat-Lösung versetzt. Danach wird bei 15 bar Wasserstoffdruck und 175-190 °C hydriert, wobei der Hydrierbeginn bei 140 °C liegt. Nach 30 min ist die Wasserstoffaufnahme beendigt.

Zur Aufarbeitung wird auf 50 °C abgekühlt, entspannt und der Katalysator abfiltriert. Das Hydrierfiltrat wird total eingedampft.

Ausbeute: 105 g Rohprodukt mit folgender Zusammensetzung:

85-90% 4-(trans-4-Butylcyclohexyl)-cyclohexanon, 5-15% 4-(4-Butylcyclohexyl)-cyclohexanol und ca. 1-5% Edukt.

Das Rohprodukt kann bei Sdp. 6 mbar 135-145 °C destilliert werden, wobei lediglich eine Reinigung von gefärbten Begleitstoffen, jedoch keine Trennung von Cyclohexanon- und Cyclohexanol-Derivat erzielt werden kann.

b) 4-(trans-4-Butylcyclohexyl)-cyclohexanon-oxim

In einer Rührapparatur werden 105,0 g obigen Rohprodukts in 800 ml Methanol gelöst und mit 30,0 g Hydroxylamin-hydrochlorid, gelöst in 30 ml Wasser, versetzt. Zu diesem Gemisch wird eine Lösung von 58,5 g Natriumacetat-Trihydrat gegeben und das Ganze 2 h unter Rückfluss verrührt, wobei das Oxim auszukristallisieren beginnt. Zur Isolierung wird auf 0-2 °C abgekühlt, das Kristallisat genutscht und mit Methanol und Wasser gewaschen. Das Feuchtkristallisat wird aus Methanol umkristallisiert.

Ausbeute: 78,6 g Oxim vom Smp: 131-132 °C, Reinheit > 99%.

Beispiel 2

4-(trans-4-Butylcyclohexyl)-cyclohexanon (Rohprodukt)

Man verfährt analog Beispiel 1, setzt jedoch bei der Hydrierung Isopropanol als Lösungsmittel ein. Die Selektivität bezogen auf das Keton beträgt 80-90%.

Beispiele 3 - 5

Analog Beispiel 1 werden folgende Verbindungen hergestellt, wobei die Reinheit jeweils > 99% beträgt

| Beispiel | R (Formel I) | Oxim Smp. °C |
|---|---|---|
| 3 | Ethyl- | 125,5 - 126 |
| 4 | Propyl- | 129 - 130 |
| 5 | Pentyl- | 131,5 - 133 |

Beispielhaft für die Verwendung der erfindungsgemässen Cyclohexanon-oxim-Derivate I werden in den nachfolgenden Ausführungsbeispielen einerseits die Spaltung zu hochreinen Ketonen II, anderseits die Reduktion zu hochreinen Aminen III beschrieben.

Beispiel 6

4-(trans-4-Butylcyclohexyl)-cyclohexanon rein

In einer 500 ml Rührapparatur werden 75,5 g 4-(trans-4-Butylcyclohexyl)-cyclohexanon-oxim, 300 ml Aceton und 7,0 ml Salzsäure 33% vorgelegt. Anschliessend wird bei 9-11 °C 90 ml wässrige Formaldehyd-Lösung (37-40%) rasch zugetropft und bei dieser Temperatur 2 h gerührt. Nachfolgend wird mit 750 ml Wasser verdünnt und mit 450 ml Toluol ausgeschüttelt. Nach der Phasentrennung wird die Toluolphase mit Wasser gewaschen und total eingedampft und das Keton im Vakuum bei 6 mbar 139-143 °C destilliert. Man erhält 66,4 g 4-(trans-4-Butyl-cyclohexyl)-cyclohexanon mit einer Reinheit von >99%: Smp.: 40-42 °C.

Beispiele 7 - 9

Analog Beispiel 6 lassen sich folgende Cyclohexanon Derivate herstellen:

| Beispiel | R (Formel II) | Sdp. °C | Smp. °C |
|---|---|---|---|
| 7 | Ethyl- | 118-122 (6 mbar) | 13 - 14 |
| 8 | Propyl- | 120-130 (3 mbar) | 40 - 42 |
| 9 | Pentyl- | 138-142 (3 mbar) | 27 - 29 |

Die Reinheit der in Beispiel 7 - 9 beschriebenen Verbindungen beträgt jeweils > 99% (GC).

Beispiel 10

4-(trans-4-Butylcyclohexyl)-cyclohexyl-amin

In einem 1 l Autoklaven werden 51 g 4-(trans-4-Butylcyclohexyl)-cyclohexanon-oxim und 400 ml 11 N Methanol-Ammoniak und 10 g Raney-Nickel eingefüllt. Anschliessend wird bei 65-85 °C und 25 bar Wasserstoffdruck hydriert. Nach ca. 20 min ist die Wasserstoffaufnahme beendigt.
Zur Aufarbeitung wird auf 20 °C abgekühlt, entspannt und vom Katalysator abfiltriert. Das Filtrat wird zur Entfernung des Ammoniaks total eingedampft. Das Konzentrat wird in 200 ml Isopropanol gelöst, mit 35 ml 5,8 N Ethanol-Chlorwasserstoff versetzt und bei 0-5 °C kristallisieren gelassen. Das Hydrochlorid wird abgenutscht und mit Ether gewaschen.
Ausbeute: 48,3 g Smp.: > 260 °C.

Die folgenden Analysen belegen die in praktischen Versuchen erzielbare hohe Reinheit.

| Für $C_{16}H_{32}$ ClN (273,89) berechnet: | | | | |
|---|---|---|---|---|
| | C 70,17, | H 11,78, | N 5,11, | Cl 12,94 |
| gefunden: | C 70,08, | H 11,73, | N 5,33, | Cl 13,12 |

Durch Behandlung mit Natronlauge kann aus dem Hydrochlorid die entsprechende ölige Base hergestellt werden.

Beispiele 11 - 13

Analog Beispiel 10 werden folgende Cyclohexyl-amin Derivate hergestellt. Die Resultate der Elementaranalyse belegen die erzielbare hohe Reinheit der Cyclohexylamin-Derivate.

| Beispiel | R (Formel III) | Hydrochlorid Smp. °C | Formel (MG) | Elementaranalyse |
|---|---|---|---|---|
| 11 | Ethyl- | > 260 | $C_{14}H_{28}$ClN (245,84) | ber.: C 68,40, H 11,48, N 5,70, Cl 14,42<br>gef.: C 68,89, H 11,13, N 6,04, Cl 14,20 |
| 12 | Propyl- | > 260 | $C_{15}H_{30}$ClN (259,86) | ber.: C 69,33, H 11,64, N 5,39, Cl 13,64<br>gef.: C 69,31, H 11,87, N 5,22, Cl 13,90 |
| 13 | Pentyl- | > 260 | $C_{17}H_{34}$ClN (287,92) | ber.: C 70,92, H 11,90, N 4,86, Cl 12,31<br>gef.: C 71,17, H 11,74, N 5,09, Cl 12,53 |

**Patentansprüche**

**1.** Cyclohexanon-oxim-Derivate der folgenden allgemeinen Formel I:

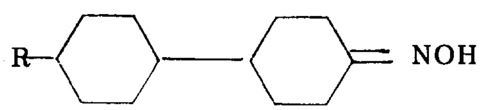

worin R eine geradkettige oder verzweigte Alkylgruppe mit 1 bis 10 C-Atomen oder eine Cycloalkylgruppe mit 3 bis 10 C-Atomen ist und worin die Bindung des Cyclohexyl-Ringes in Bezug zum Substituenten R sowohl in cis- als auch in trans-Stellung konfiguriert sein kann.

**2.** Cyclohexanon-oxim-Derivate gemäss Anspruch 1, dadurch gekennzeichnet, dass der Substituent R eine geradkettige oder verzweigte Alkylgruppe mit 1 bis 7 C-Atomen oder eine Cycloalkylgruppe mit 4 bis 7 C-Atomen ist.

**3.** Cyclohexanon-oxim-Derivate gemäss Anspruch 2, dadurch gekennzeichnet, dass der Substituent R eine Methyl-, Ethyl-, Propyl-, Butyl-, Pentyl-, Hexyl- oder Heptyl-Gruppe oder eine Isopropyl-, Isobutyl-, Isopentyl-, Isohexyl- oder Isoheptyl-Gruppe in allen möglichen isomeren Formen ist.

**4.** Cyclohexanon-oxim-Derivate gemäss Anspruch 2, dadurch gekennzeichnet, dass der Substituent eine Cyclobutyl-, Cyclopentyl-, Cyclohexyl- oder Cycloheptyl-Gruppe ist.

**5.** Cyclohexanon-oxim-Derivate gemäss Anspruch 2, dadurch gekennzeichnet, dass die Bindung des Cyclohexyl-Ringes in Bezug zum Substituenten R trans-konfiguriert ist.

**6.** Verfahren zur Herstellung von Cyclohexanon-oxim-Derivaten gemäss einem der Ansprüche 1 bis 5, dadurch gekennzeichnet, dass man alkyl- oder cycloalkylsubstituierte Cyclohexylphenole der folgenden allgemeinen Formel IV:

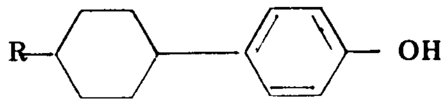

worin R die in den Ansprüchen 1 bis 5 angegebene Bedeutung hat und die Bindung des Phenyl-Ringes in Bezug zum Substituenten R cis- oder trans-konfiguriert sein kann, einer selektiven katalytischen Hydrierung bei 5 bis 40 Bar, bevorzugt bei 15 Bar Wasserstoffdruck, 175 bis 200 °C, während einer Reaktionsdauer von 10 bis 60, bevorzugt 30 Minuten, und unter Verwendung eines nach dem Stand der Technik üblichen Katalysators und Lösungsmittels unterwirft, das im resultierenden Reaktionsgemisch dieser Phenol-Hydrierung vorhandene Keton zum Oxim umsetzt und das Oxim zur Reinigung isoliert.

**7.** Verfahren gemäss Anspruch 6, dadurch gekennzeichnet, dass man das nach der katalytischen Hydrierung vorliegende Rohgemisch in einem Lösungsmittel löst und mit Hydroxylamin oder dessen Salzen, mit anorganischen oder organischen Säuren, vorzugsweise Hydroxylamin-sulfat, Hydroxylamin-hydrochlorid, Hydroxylamin-phosphat, Hydroxylamin-acetat umsetzt.

**8.** Verfahren gemäss den Ansprüchen 6 oder 7, dadurch gekennzeichnet, dass man als Lösungsmittel für die Umsetzung niedere Alkohole wie Methanol, Ethanol, Propanol und Isopropanol oder Gemische dieser Alkohole mit wechselnden Mengen Wasser einsetzt.

**9.** Verfahren zur Herstellung von hochreinen Cyclohexanon-Derivaten der folgenden allgemeinen Formel II:

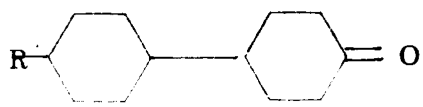

worin R die in den Ansprüchen 1 bis 5 angegebene Bedeutung hat und die Bindung des Cyclohexyl-Ringes in Bezug zum Substituenten R cis- oder trans-konfiguriert sein kann, aus einem Cyclohexanon-oxim-Derivat gemäss einem der Ansprüche 1 bis 5, dadurch gekennzeichnet, dass man das nach dem ersten Reaktionsschritt gemäss einem der Ansprüche 6 bis 8 resultierenden isolierte Oxim in einem zweiten Reaktionsschritt durch saure Hydrolyse, oxidative Spaltung oder durch Verdrängung durch reaktionsfähigere Carbonylgruppen zum Cyclohexanon-Derivat umsetzt und anschliessend gegebenenfalls reinigt.

**10.** Verfahren zur Herstellung von hochreinen Cyclohexylamin-Derivaten der allgemeinen Formel III:

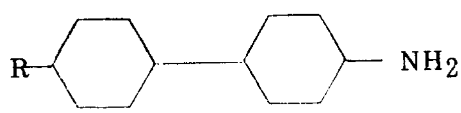

worin R die in den Ansprüchen 1 bis 5 angegebene Bedeutung hat und die Bindung des Cyclohexyl-Ringes in Bezug zum Substituenten R cis- oder trans-konfiguriert sein kann, sowie gegebenenfalls deren Salzen mit anorganischen oder organischen Säuren aus einem Cyclohexanon-oxim-Derivat gemäss einem der Ansprüche 1 bis 5, dadurch gekennzeichnet, dass man das nach dem ersten Reaktionsschritt gemäss einem der Ansprüche 6 bis 8 resultierende isolierte Oxim nachfolgend in einem zweiten Reaktionsschritt in Gegenwart von Ammoniak katalytisch hydriert und in an sich bekannter Weise reinigt und gegebenenfalls die Cyclohexylamin-Derivate III in ihre Salze mit anorganischen oder organischen Säuren überführt.

**11.** Verfahren gemäss Anspruch 10, dadurch gekennzeichnet, dass man den zweiten Reaktionsschritt bei 50 bis 100 °C und bei 5 bis 40 Bar Wasserstoffdruck, bei einer Reaktionsdauer von 10 bis 60,

bevorzugt 20 Minuten durchführt.

**12.** Cyclohexylamin-Derivate der allgemeinen Formel III, worin R eine geradkettige oder verzweigte Alkylgruppe mit 1, 2 oder 4 bis 10 C-Atomen oder eine Cycloalkylgruppe mit 3 bis 10 C-Atomen ist und die Bindung des Cyclohexyl-Ringes in Bezug zum Substituenten R cis- oder trans-konfiguriert sein kann, sowie deren Salze mit anorganischen oder organischen Säuren.

**13.** Cyclohexylamin-Derivate gemäss Anspruch 12, dadurch gekennzeichnet, dass R eine geradkettige oder verzweigte Alkylgruppe mit 1, 2 oder 4 bis 7 C-Atomen oder eine Cycloalkylgruppe mit 4 bis 7 C-Atomen ist.

**14.** Cyclohexylamin-Derivate gemäss Anspruch 13, dadurch gekennzeichnet, dass R eine Methyl-, Ethyl-, Butyl-, Pentyl-, Hexyl- oder Heptyl-Gruppe oder eine Isopropyl-, Isobutyl-, Isopentyl-, Isohexyl- und Isoheptyl-Gruppe in allen möglichen isomeren Formen ist.

**15.** Cyclohexylamin-Derivate gemäss Anspruch 13, dadurch gekennzeichnet, dass R eine Cyclobutyl-, Cyclopentyl-, Cyclohexyl- oder Cycloheptyl-Gruppe ist.

**16.** Cyclohexylamin-Derivate gemäss Anspruch 12, dadurch gekennzeichnet, dass die Bindung des Cyclohexyl-Ringes in Bezug zum Substituenten R trans-konfiguriert ist.